# EUROPEAN PATENT SPECIFICATION

(11) **EP 2 941 178 B1**
(45) Date of publication and mention of the grant of the patent: **23.10.2019**
(21) Application number: 13818673.9
(22) Date of filing: 30.12.2013
(51) Int. Cl.: A61B 3/12, A61F 9/008, A61B 3/10, A61B 3/14, G06K 9/00, G06T 7/00

(54) **SYSTEM AND APPARATUS FOR PROVIDING OPHTHALMIC IMAGES FOR DIAGNOSIS AND TREATMENT**
SYSTEM UND VORRICHTUNG ZUR BEREITSTELLUNG VON AUGENABBILDUNGEN ZUR DIAGNOSE UND BEHANDLUNG
SYSTÈME ET APPAREIL DESTINÉS À FOURNIR DES IMAGES OPHTALMIQUES POUR LE DIAGNOSTIC ET LE TRAITEMENT

(30) Priority: 04.01.2013 US 201361749107 P
(43) Date of publication of application: 11.11.2015
(73) Proprietor: Quantel Medical, 63800 Cournon-D'Auvergne (FR)
(72) Inventor: CHABRIER, Christian, 63670 La Roche Blanche (FR); LETOFFET, Lionel, 63450 Saint-Amant-Tallende (FR); SCHNITZLER, Jean-Yves, 63000 Clermont-Ferrand (FR); WASSMER, Benjamin, 63000 Clermont-Ferrand (FR)
(74) Representative: Regimbeau
(86) International application number: PCT/EP2013/003949
(87) International publication number: WO 2014/106536

(56) References cited:
- WO-A1-2008/033933
- US-A1- 2009 093 798
- US-A1- 2009 163 898
- US-B1- 6 454 410

## Description

### FIELD OF THE INVENTION

The invention relates to the diagnosis and treatment of ophthalmic diseases in general, in particular retinal diseases that rely heavily on photographic and angiographic imaging of the eye.

### BACKGROUND OF THE INVENTION

Generating good quality ophthalmic images are vital for clinical care in preventing and treating the most common causes of vision loss, including diabetic retinopathy and age-related macular degeneration. Photographic and angiographic images facilitate discovery of ophthalmic abnormalities by documenting changes to the eye over time. For example, abnormalities may be discovered in real-time by comparing images taken during a patient examination with previously taken photographic images.

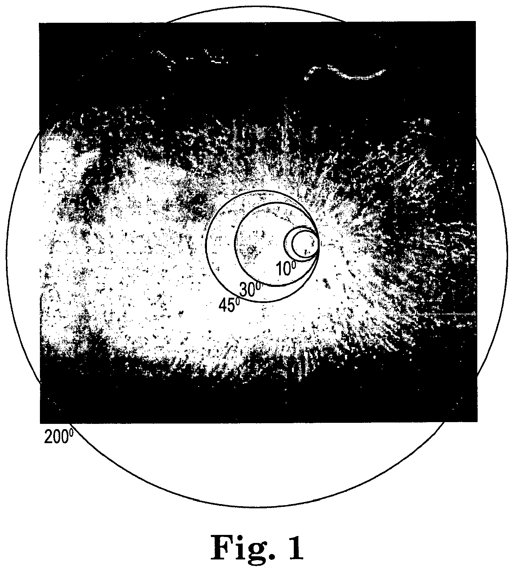

By way of background, a retina 10 could be separated in two mains parts (see FIG. 1): the central area (macula) 12 inside the inner circle while the periphery 14 is outside the inner circle. In the retina, different areas are shown with the corresponding angle of view, with the various concentric circles illustrating views ranging for the user from 10° to 200°. The following are examples of prior art treatments that rely on imaging technology for effective treatment of the patient.

Diabetic Retinopathy: In a diabetic patient, the disease alters the vascularization of the retina that could lead to blindness. In this case, the retina needs to be partially coagulated in order to limit the extent and effect of the disease on a patient's eyesight. For this pathology, one of the most popular treatments is laser photocoagulation wherein a laser beam is projected through the eye towards the retina. The wavelength of the laser is chosen so as to not be absorbed by the different eye medium or tissue layers (cornea, vitra, lens...) before reaching the retina. Hence the laser energy that reaches the retina is absorbed and the resulting heating is enough to coagulate the retina tissue locations.

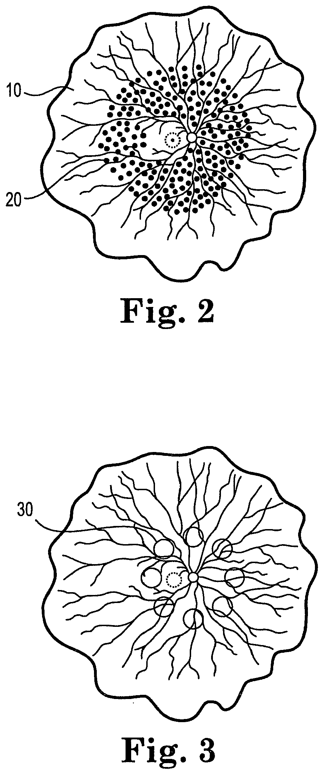

Pan Retinal Photocoagulation (PRP): This type of laser treatment can be performed in the whole retina (central area and periphery), however the central area is treated today mostly by chemical agents and not by laser photocoagulation. In extreme cases, the laser could be used but each laser shot will permanently impact the patient's vision and such will be readily evident to the patient. Outside of the periphery, use of a laser for treatment is standard in the industry. To treat the whole periphery, the number of laser shots 20 on retina 10 are important and necessary to have an effective outcome (See FIG. 2). Typically, this PRP is realized in an angle between 45° to 160° (see FIG. 1).

Referring to FIG. 3, before the introduction of the multispot laser, a physician had to press the treatment system footswitch each time he wanted to actuate the laser and treat a patient's eye tissue. Hence due to the high number of shots during a PRP procedure, the procedure would be very slow and tedious. To accelerate the process of PRP, scanners were introduced in order to realize multispot treatment 30. The physician chooses the pattern he wants to deliver (circle, rectangular, line, arc...) the number of points or spots and the interval between those spots. As for the monospot laser, an aiming beam is emitted before firing to show where the treatment laser will be positioned.

The first lasers introduced for eye treatment applications were so powerful such that the energy used resulted in carbonization of the retina. With the second generation of lasers, the energy was reduced by reducing or shortening the exposure time from 20 ms to 5 ms whereas before it was at 50 to 200 ms. However the laser spots still marked or damaged the retina. To treat more specifically the macula, in case of edema, the latest generation of lasers use a micropulse firing mode (a train of pulses at 100 µs to 500 µs) to have just enough energy to treat the retina without leaving a visible mark or damage to surrounding tissue. In these laser treatment systems, the aiming beam is typically a low power laser (less than 1 mW) at a wavelength visible (for example 532 nm) by the physician but different than for the treatment beam (for example 635 nm). The physician can see the aiming beam but he is protected by protective eyewear in case the treatment beam accidently fires towards his own eye. The aiming beam has no effect on the tissue as it is just used to indicate where the treatment laser will be fired. The treatment beam on the other hand has more power (between 0.1 to 3 W) with a wavelength of about 577 nm in order to obtain a photocoagulative effect on the selected eye tissue.

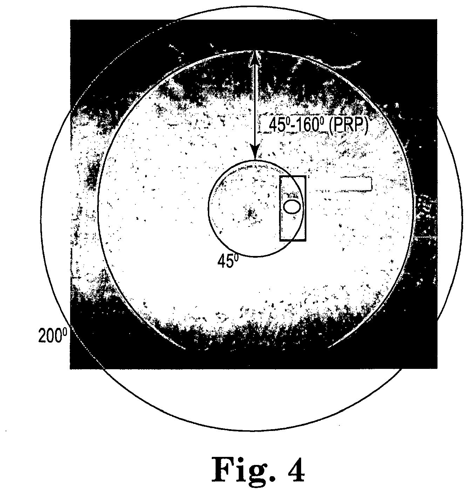

One of the devices used to diagnose a patient's eye or for firing a laser into a patient's eye is a slit lamp. The slit lamp is an ophthalmic diagnosis tool that combines a binocular microscope with a lighting slit focused on the patient retina. This device could be used with or without a contact lens. In treating the eye, the laser is mounted such that the beam is collinear to the slit lamp, which allows the physician to control the position of the laser. The slit lamp, however, only provides a very narrow field of view, as seen in a black rectangle in FIG. 4. This forces the physician to move the lamp around (slit image after slit image) in order to have a complete view of the retina, with the physician never having a complete view of the retina. A direct ophthalmoscope and slitlamp biomicroscope, on the other hand, are instruments that are common to every examination room in an ophthalmologist's and optometrist's office. Furthermore, these instruments may be attached to an image acquisition device, like a charge coupled device (CCD) camera. The CCD camera in combination with the direct ophthalmoscope and slitlamp can acquire images of the back of the eye for documentation and communication purposes. However, because the direct ophthalmoscope and biomicroscope provide a reduced image quality and a far narrower field of view (as compared to the fundus camera), the images they provide are not useful for photodocumentation.

Video cameras have also been mounted on slit lamps to facilitate taking pictures or displaying what the physician sees for educational purposes, however the angle view of the camera is identical to that of the physician's angle of view through the slit lamp. With respect to still images, photographic images of the fundus (back of the eye) or retina have been acquired in the past with a standard fundus camera, available commercially from Carl Zeiss, Inc., model number FF450. The fundus camera provides a high quality, wide-field of view image of the fundus. However, because of its high cost and the fact that it is a dedicated instrument, offering no use other than photography, many optometrists and ophthalmologists opt not to have a fundus camera in their office. Therefore, fundus photography, is rarely performed during the routine examination that a majority of patients undergo.

Presently, image processing techniques exist that construct mosaic representations from individual still and video imagery. For example, U.S. Patent No. 5,684,561 to Yancey describes creating a create a mosaic image of the retina with multiple images (e.g., col. 15, line 62), while in U.S. Patent No. 5,912,720 to Berger et al. there is described a CCD camera in use with a slitlamp to create a mosaic image. However, some of the challenges faced with creating mosaic images of the retina and fundus have to do in part with narrow field of view, rapid movement of the subject eye, specular reflections, areas of low feature contrast, and geometric image distortion. In U.S. Patent No. 6,454,410 to Berger et al there appears to be described a mosaic of the retina being made from a narrow acquisition device, such as a regular camera with a slit lamp (or a direct ophthalmoscope). However, it appears that this system may have its limitations in terms of other image acquiring devices with which it can be used.

### BRIEF SUMMARY OF THE INVENTION

Even though the main diagnostic tool for an ophthalmologist today still appears to be the slit lamp, one of its major weaknesses is the inability to record accurately a global image of the retina. An advantage of the diagnostic and treatment systems described herein, is the ability to build a retinal image or a mosaic image primarily using a slit lamp as well as the capability of having a retinal image that extends far into the periphery. In one example embodiment, the diagnostic/treatment system described herein has one or more of the following capabilities and uses: 1) with the slit lamp and the laser combination we use the image provided by the slit lamp to prepare the treatment plan; and 2) the system is able to import images provided by third party diagnostic devices. These diagnostic images are usable to prepare the treatment plan instead of using the mosaic-formed slit lamp image, and/or are usable to overlay such images onto the slit lamp image(s). The scope of the invention is defined by the independent claim, preferred embodiments are described in the dependent claims. Any subject-matter described in the following, which falls out of the scope of the claims is presented for illustrative purposes only.

In an example embodiment, a system for generating ophthalmic images in real time for the treatment of eye diseases includes an eye imaging device which acquires a real-time image of a retina of a patient for visualization by a user and a video image module operatively coupled to said eye imaging device, the video image module including a video capturing device and a video processing unit, the video capturing device adapted to capture real-time video images of the retina. The system further includes an image processing module operatively coupled to the video image module and configured to generate a reference image of the retina from the real-time video images of the retina and an image recombination module operatively coupled to the image processing module and configured to overlay at least one secondary image over the reference image received from at least one secondary ophthalmic diagnostic device. The system also includes a means for localizing and centering said eye imaging device in real time based on the reference image of the retina, localizing means operatively coupled to the image processing module. In a related embodiment, the video processing unit is configured to perform at least one of the operations from a group consisting of slit extraction, slit localization or centering, image reflection correction, image validation and image selection from at least one camera. In yet another related embodiment, the reference image is selected from the group consisting of a mosaic image, an image from the secondary imaging device and a secondary ophthalmic diagnostic device. The at least one secondary image is also selected from the group consisting previously stored photographic, retinographic, angiographic and OCT angiographic images. The eye imaging device of this system can include at least one of a slit lamp, an indirect ophthalmoscope and a fundus camera, and OCT imaging. This system can also include an aiming beam adapted for calibrating and verifying a scanner device position through the localization of an aiming beam spot on the reference image.

In yet another related embodiment of this system for generating ophthalmic images, the system further includes means for generating at least one of an ophthalmic diagnostic plan and a laser treatment for the patient operatively coupled to said image recombination module. The image processing module comprises software which permits interactive control of at least one of image selection, pointing, and annotation by the user. The image recombination module is configured to overlay at least one secondary image from the secondary diagnostic device on a live slit lamp image of the patient thereby generating an augmented reality image. In a related embodiment, the system further includes a laser system and scanning device adapted to be controlled in response to the augmented reality image and are localized as part of the treatment plan. The system further includes an aiming beam adapted for calibrating the reference image that is being generated.

In another example embodiment, a system for generating ophthalmic images in real time and for the treatment of eye diseases includes an eye imaging device which acquires a real-time image of a retina of a patient for visualization by a user and a video image module operatively coupled to said eye imaging device, the video image module including a video capturing device and a video processing unit, the video capturing device adapted to capture real-time video images of the retina. The system also includes an image processing module operatively coupled to the video image module and configured to generate a reference image of the retina from the real-time video images of the retina and includes means for localizing and centering the eye imaging device in real time based on the reference image of the retina, localizing means operatively coupled to the image processing module. The system further includes means for generating an ophthalmic treatment plan for the patient operatively coupled to the image recombination module and a laser system and scanning device that are localized and centered in response to the treatment plan. In a related embodiment, if the laser treatment is interrupted during the pattern treatment, a software module will indicate to the user which spots remain of the pattern for treatment (so as not to re-treat some areas and not to lose track of where the user is on the patient's retina).

In a related embodiment to this system, there is also included an image recombination module operatively coupled to the image processing module and configured to overlay at least one secondary image over the reference image received from at least one secondary imaging device, thereby generating an augmented reality image. The video processing unit is configured to perform at least one of the operations from a group consisting of slit extraction, slit centering, image reflection correction, image validation and image selection from at least one camera. This system can also include an aiming beam adapted for calibrating the reference image that is being generated. The image processing module of this system is also configured to have a traceability function such that the reference image of the patient is updated with an image of the retina after a last laser shot or updated with the position of the laser shot. The image processing module is configured to have an extended localization function such that the reference image of the patient is updated with an image of the retina after a previous laser treatment to extend the reference image.

In yet another example embodiment, a system for generating ophthalmic images in real time and for the treatment of eye diseases includes an eye imaging device which acquires a real-time image of a retina of a patient for visualization by a user and a video image module operatively coupled to the eye imaging device, the video image module including a video capturing device and a video processing unit, the video capturing device adapted to capture real-time video images of the retina. The system also includes a traceability module operatively coupled to the video module and adapted to update a retinal image of the patient, the retinal image update selected from the group consisting of an image of the retina after a previous laser firing, an updated position of the laser shot on the retinal image and an updated position of the laser. The system can also include localization means operatively coupled to said traceability module and adapted to extend the traceability function to a larger image, wherein with the reference image is a mosaic image.

In yet another example embodiment, a system for generating ophthalmic images in real time and for the treatment of eye diseases is provided that includes an eye imaging device which acquires a real-time image of a retina of a patient for visualization by a user and a video image module operatively coupled to said eye imaging device, the video image module including a video capturing device and a video processing unit, the video capturing device adapted to capture real-time video images of the retina. The system also includes an image processing module operatively coupled to the video image module and configured to generate a reference image of the retina from the real-time video images of the retina. The system also includes an aiming beam module including an aiming beam operatively coupled to the image processing module and adapted to project at least one spot on the retina and to display to a user a pattern of spots on either a live retinal image or the reference image.

In an embodiment related to the alignment function above, the aiming beam module is configured to: either fill a free formed area on the retinal image with one or more spots prior to a treatment; not to energize the aiming beam of the entire pattern of spots but only on at least one spot localized or centered on the retina prior to a treatment; or to provide at least one spot within the pattern of spots defined as an untreated spot, said untreated spot being defined to the user by a signal selected from the group consisting of a blinking spot, a spot of a different color, a spot of changing colors, and a spot of varying dimension or size or shape (or have a different behavior than the other spots within the aiming beam pattern). For example, a blinking spots indicates that this spot will not be treated. In a 3x3 pattern, the user would decide not to treat the central point, hence this central point will have a different behavior than the others points of the pattern. One advantage of having a blinking behavior on one spot is that the physician can see the retina when the intensity is off.

In yet another example embodiment, a method for generating ophthalmic images in real time for the treatment of eye diseases is provided that includes acquiring real-time images of a retina of a patient through a slit-lamp device and video recording the images and generating a mosaic image of the retina from the real-time video images of the retina. The method also includes the step of centering or localizing or aligning the slit lamp in real time based on the mosaic image of the retina and then generating an ophthalmic diagnostic plan for the patient. In a related embodiment, the method also includes the step of overlaying images over the mosaic image received from at least one other ophthalmic diagnostic device, thereby generating an augmented reality image. This method can also include the step of guiding a laser during laser therapy of the eye using the augmented reality image. In a related embodiment, the method includes the step of using an aiming beam and associated spots to perform one of calibrating the distortion in forming the mosaic image, adjusting the focus of the laser treatment, adjusting the size of the laser spot and as an indication of the percentage of magnification zoom being used.

In another embodiment, the diagnostic/treatment system described herein has the capability to mark-up the mosaic image in order to prepare a treatment plan; exhibit with an aiming beam the point where the laser will be fired; fire a laser shot on a predefined or premarked point or spot, and stop the laser shot/firing if the current localization or selected treatment target is not on the treatment plan. In a related example embodiment, a system and a diagnostic tool is provided that is comprised of a hardware portion or module, including a slit lamp, a video camera, a computer, a screen and a laser, and a software portion or module which will facilitate an interface between the hardware or module and a Human-Machine Interface (HMI).

The invention now will be described more fully hereinafter with reference to the accompanying drawings, which are intended to be read in conjunction with both this summary, the detailed description and any preferred and/or particular embodiments specifically discussed or otherwise disclosed. This invention may, however, be embodied in many different forms and should not be construed as limited to the embodiments set forth herein; rather, these embodiments are provided by way of illustration only and so that this disclosure will be thorough, complete and will fully convey the full scope of the invention to those skilled in the art.

### BRIEF DESCRIPTION OF THE DRAWINGS

Other important objects and advantages of the present invention will be apparent from the following detailed description of the invention taken in connection with the accompanying drawings in which;
FIG. 1 illustrates a retina which is separated into the macula (inside the circle) and the periphery that is outside the macula, and illustrating various zones of visibility and treatment (such as between 10° to 200°).
FIG. 2 illustrates a retina having had a multispot laser with an aiming beam is emitted before firing to show where the laser position.
FIG. 3 illustrates a multispot pattern formed on a retina by an aiming beam that is emitted before treatment laser firing and to indicate where treatment will take place.
FIG. 4 illustrates a retina where a slit lamp is able to see only a portion of the retina shown in a black rectangle.
FIG. 5 illustrates an example embodiment of a diagnostic tool and treatment system according to the teachings of the invention.
FIG. 6 illustrate another example embodiment of a diagnostic system according to the teachings of the invention.
FIG. 7 illustrates yet another example embodiment of a system and a diagnostic tool and treatment system according to the teachings of the invention. In this example embodiment, the Localization module/step represents where only the spot(s) inside the current slit are shown or pointed with the aiming beam of the system.
FIGS. 8A-8B illustrate stereo acquisition and recombination of retinal images according to the teachings of the invention.
FIGS. 9A-9B illustrate an example of slit lamp extraction wherein a number of images filters will be implemented to extract only the useful area from the whole images taken of the retina.
FIG. 10 is an image illustrating image distortion characterization using an aiming beam. The rectangle with the dotted line is the preset pattern without being distorted while the solid line is the pattern distorted.
FIG. 11 is a photographic representation of a mosaic image of a retina generated by the diagnostic system taught herein.
FIGS. 12A-12B are various photographs of a retina periphery illustrating areas with a visible laser shots (which are then joined together to form a mosaic image) for use in extended localization diagnosis or treatment according to the teachings of the invention.
FIG. 13 illustrates an example of a mosaic creation screenshot.
FIG. 14 illustrates preparation of a treatment plan screenshot with a standard treatment pattern.
FIG. 15 illustrates a screenshot of a laser treatment plan according to the teachings herein.
FIG. 16 illustrates a screenshot of a laser treatment plan allowing a user to select a free area grid instead of a pattern according to the teachings herein.
FIG. 17 illustrates a screenshot of a laser treatment on a grid according to a treatment plan as taught herein.

### DETAILED DESCRIPTION OF THE INVENTION

Following below are more detailed descriptions of various related concepts related to, and embodiments of, methods and apparatus according to the present disclosure for an improved diagnostic and treatment system that speeds up eye treatment time while improving accuracy and reliability of the selected treatment by the physician. It should be appreciated that various aspects of the subject matter introduced above and discussed in greater detail below may be implemented in any of numerous ways, as the subject matter is not limited to any particular manner of implementation. Examples of specific implementations and applications are provided primarily for illustrative purposes.

In one example embodiment, the diagnostic/treatment system described herein has one or more of the following capabilities and uses: 1) with the slit lamp and the laser combination we use the image provided by the slit lamp to prepare the treatment plan; and 2) the system is able to import images provided by third party diagnostic devices. These diagnostic images are usable to prepare the treatment plan instead of using the mosaic-formed slit lamp image, and/or are usable to overlay such images onto the slit lamp image(s).

In another example embodiment, there is described a diagnostic and treatment device comprised of a slit lamp, a laser and an imaging device such as angiography or an OCT angiography to focus on the areas to be treated. A slit lamp by itself provides too small of a view of the retina and forces the physician to constantly return to the macula to get his bearings in the periphery of the retina and compare that with the image he is working from (for example, a print out) to check on the status of the treatment. Hence integrating other imaging devices helps to improve physician's view of the patient's eye, leading to improved diagnosis and treatment. This is especially true with OCT which can help to see and focus on the areas to be treated inside the retina.

In treating the patient, the physician strives not to accidently fire a laser shot in the macula and constantly checks on parameters such as laser power, shot duration, diameter of the spot and the pattern (form, number of spot, interval between spot, etc.). Image quality coming from imaging devices can also affect diagnosis and treatment. Some factors that impact the quality of the images from the imaging devices include the size of the retina, the zoom used (which is adjustable at any time by the physician) and slit size/shape and intensity (which are in turn affected by patient pain, the cornea clarity, the pupil dilatation, eye lens disease (such as a cataract), the retina texture and color, the contact lens position on eye) and slit lamp reflection (typically the white rectangle in previous pictures).

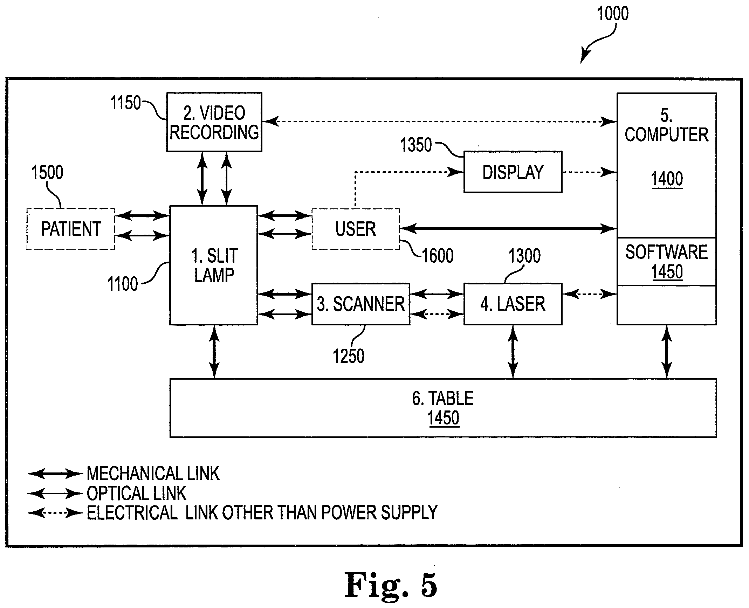

One of the aims of this invention is to integrate hardware components/modules and software components/modules into a diagnostic tool and treatment system speeds up treatment time while improving accuracy and reliability of the selected treatment by the physician. Referring now more specifically to FIG. 5, there is shown an example embodiment of a diagnostic tool and treatment system 1000 comprised of a slit lamp 1100, a video imaging device 1150 (including but not limited to a video camera), a scanner 1250, a laser 1300, a viewing device or screen 1350, a computing device 1400 and a database or table 1450. The various components are interconnected as shown via a series of mechanical links, optical links or electrical links (other than the power supply to each of the components). A software module 1450 operates to facilitate a Human-Machine Interface (HMI) with the rest of system 1000. A patient 1500, with the help of user 1600, interacts with system 1000 to determine an eye health diagnosis, an eye treatment via the laser or a health diagnosis (of one of more disease states) with subsequent laser or chemical treatment shortly thereafter.

In a related embodiment, scanner 1250 and laser 1300 are omitted so that system 1000 operates primarily as a diagnostic tool. In most systems today, the laser is collinear to the central axis of the viewer of the slit lamp. In coordination with the scanner, the beam is moved around this central axis to illustrate some pattern spots. The maximum displacement of the scanner is limited to a few millimeters. However, the width of the slit or the visible area could be lower than the scanner displacement. Hence, system 1000 can limit the scanner displacement to the accessible area.

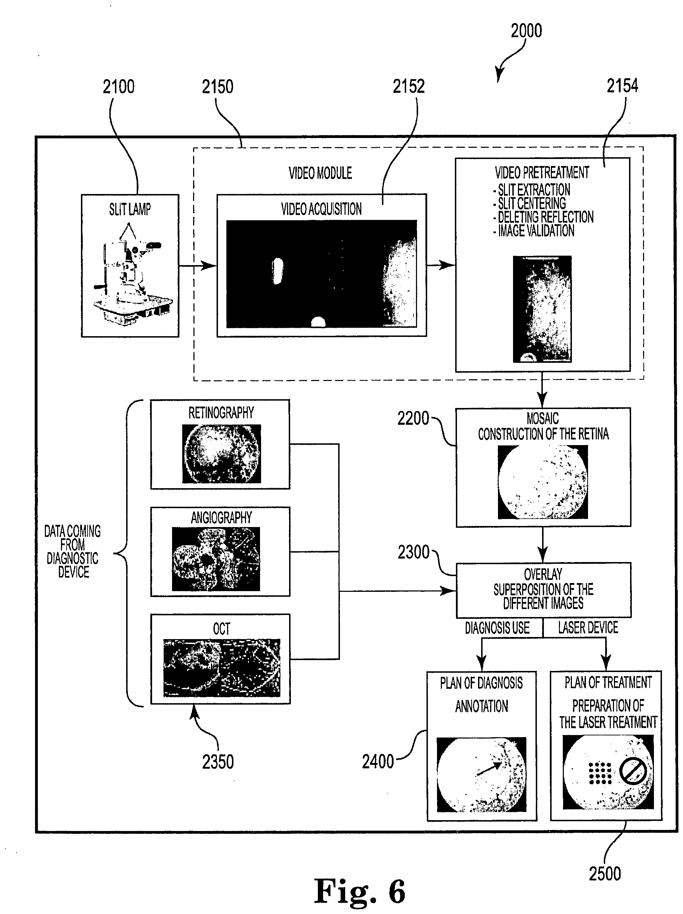

Referring now to FIG. 6, there is shown another example embodiment of a diagnostic tool 2000 comprised of a slit lamp 2100, a video module 2150, a MOSAIC construction module 2200, and an image recombination (Overlay) module 2300 that receives image(s) from another diagnostic device 2350 to thereby generate a diagnosis plan 2400 or a treatment plan 2500 for the user or physician. In this example embodiment, video module 2150 includes a video acquisition device 2152 that provides images to a video pretreatment module 2154 that facilitates slit extraction and centering and is able to delete reflections and provide image validation prior to serving as an input to construction module 2200.

With system 2000, a physician (being assisted by integrated software) will be able to: read images and data coming from other diagnostic device 2250, which can be one or more of retinography, angiography or OCT angiography; and if necessary, build the image of the retina (MOSAIC) via construction module 2200. Before doing any patient procedure, system 2000 will overlay the different images and data and prepare a diagnosis plan which could be composed of an annotation of the vein to be carefully explored, or the area to be carefully examined. As part of the Overlay (image recombination) function, the software will integrate different functions to automatically position the diagnostic images together. In a related embodiment, manual positioning is used to position the images together. In yet another embodiment, OCT imaging is used for capturing an image of the retina and perform a similar process as with the slit lamp. This feature provides the advantage of coupling the laser treatment with OCT imaging as OCT has the advantage of generating 3D images instead of 2D images.

Where the procedure requires use of a laser for treating retinal tissue, system 2000 generates a treatment plan which will consist of indicating the position of the laser spots that have to be performed, and/or the position of the area to be excluded from treatment (such as the macula, optical nerve...). In one example embodiment, the treatment plan includes one or more of the following: position of the laser shot, the area to be excluded from treatment, the size of the laser spot, and the distance between spots. Today the laser devices could be used in monospot or in predefined pattern spot. In another example embodiment, the treatment plan generated by the system 1000 or 2000 first selects a large area and then is filled in with randomized placement of a spot or spots. Because the treatment plan can be prepared using the diagnostic images, treatment preparation can proceed without the patient having to sit and wait in front of the treatment system. Upon the patient's arrival, the treatment plan is uploaded to the treatment system and the associated images will be superimposed (or overlayed) on the live image of the patient.

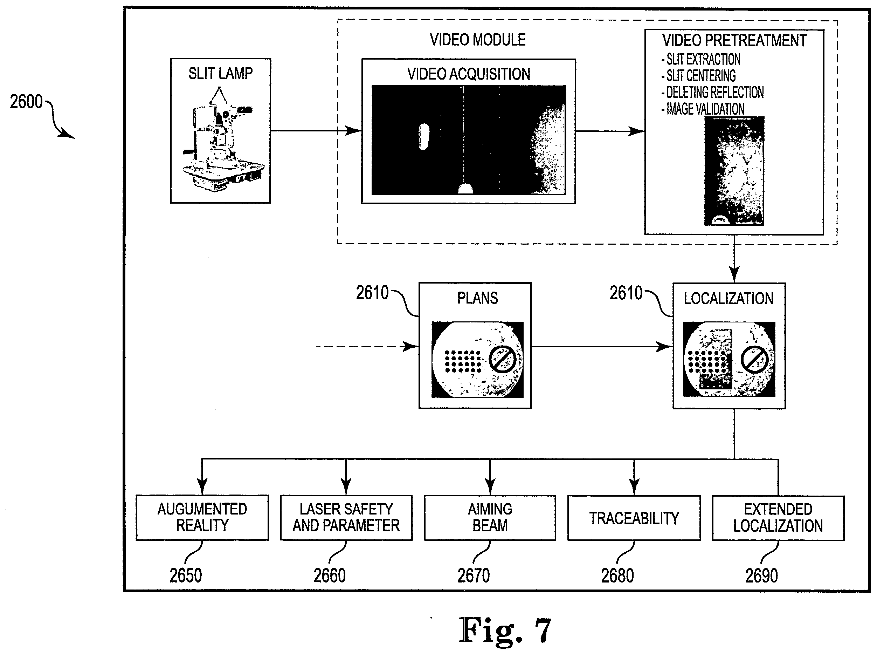

Referring now to FIG. 7, there is illustrated another example embodiment of a diagnostic system and treatment system 2600 according to the teachings of the invention. System 2600 takes system 2000 to another level with more functionality, such as a Localization module 2610 (and part of a processing step) represents where only the spot(s) inside the current slit are shown or pointed with the aiming beam of the system during performance of either plan (diagnosis or treatment), the slit position and its view is affected by the overlay on the live slit image either the diagnostic data or the plan of treatment (PLANS module 2610) thereby generating an augmented reality (AUGMENTED REALITY module 2650). More specifically, Augmented Reality 2650 functions to superimpose other pictures or images provided by any one of a number of diagnostic devices onto the current slit lamp images, which will then be displayed on the computer screen. A diagnostic device is located close to the slit lamp to allow the physician to look through the binocular of the slit lamp and with as little movement as possible to view the screen. For this application, the physician could have a live image and then choose to overlay with other diagnostic images. In a related embodiment, the laser treatment plan is one part of the augmented reality and can be considered as one image that could be optionally overlayed on the live retinal image along with other images coming from other diagnostic devices.

The diagnostic system 2600 describe herein also provides a slit lamp Extended Localization 2690 function or capability. Currently there are no devices that will localize or align current slit lamps with the retina, which can lead to generating erroneous retinal images. It is also fairly easy to misalign the slit lamp since the image being viewed is inverted due to the use of the indirect contact lens and the position of the optic nerve and central area of the retina changes depending if you look at the right eye or left eye of the patient. Therefore, with the system described herein localization is made in real time based either on the mosaic image previously generated or on other diagnostic images, thereby reducing the likelihood of producing incorrect images. In case of very low laser power or the use of the micropulse laser treatment mode, the positions of the laser shots are invisibles during and after the treatment. Hence, the combination of the localization mode/mean with the laser device provides data on where on the retina the laser has been fired or not (to ensure one spot does not get treated twice) and data on where the spots to be treated are too close (for example). The minimum spot diameters typically used today are around 100 µm because the movement of the patient and physician does start to present challenges when you pass below this spot diameter. However, due to the precision and the localization of the diagnostic/treatment systems described herein, treatments with spot sizes as small as 50 µm are possible, especially in the central area with or without the use of the zoom feature to accurately fire the laser shot on the desired spot. For purposes of traceability, the position of this non-visible spot will be stored relating to the retina images. In some cases, use of micropulse treatment mode or multiple treatments on the same area/point will now be possible due to the highly accurate data now available to the physician of the previous treatment location provided by the systems described herein.

In this example embodiment, system 2600 automatically detects if the current position that is being viewed is one where a laser shot is to be performed or not. In the latter case, the laser will be disconnected to prevent any inadvertent or accidental laser firing (indicated as the function of LASER SAFETY 2660). In a related embodiment, laser safety is implemented by disarming the laser or using an alarm (or other sound) to indicate whether or not the slit lamp is positioned in the laser area. In yet another related embodiment, the laser safety function is also used if a movement of the patient or operator occurs that could lead to an unplanned or accidental laser firing. The laser parameter could be also automatically adjusted as a function of the slit lamp position and the plan of treatment.

Referring again to FIGS. 5-7, the aiming beam, as part of the diagnostic/treatment system, indicates the spot or spots to be treated according to the treatment plan (AIMING BEAM 2670). Hence, the aiming beam could be managed by the localization function and then only the spot intended to be treated could be indicated by the aiming beam. Moreover, if the laser is interrupted during the selected treatment pattern, the software will see the remaining points/spots and will indicate same using the aiming beam. In this example embodiment, the aiming beam has a wavelength of about 635 nm while a treatment beam has a wavelength of about 532 nm. In a related embodiment, the aiming beam is configured to determine and verify the scanner position through the localization or centering of the aiming beam spot on the image.

The system described herein also provides for aiming beam modulation such that an aiming beam spot, visible on the image from the camera, is used for calibrating and checking that the scanner is functioning. An optic filter can be added in front of the camera for decreasing the aiming beam intensity. By synchronizing the aiming beam and the acquisition of one image, during one image the intensity of the aiming beam is put at the maximum level so as to be visible through the filter for calibrating the scanner position. The rest of the time, the intensity of the aiming beam will be too small and will not be visible on the camera. The user may also modulate the aiming beam intensity, inside a pattern, such that one position or spot is brighter than others. For example, the first position will be the one which is brighter for informing the physician of the first laser shot position.

In a related embodiment, modification of spectral range of the laser is with an infrared source and a sensor between 700nm and 900 nm, which allows a decrease of the amount of light to reduce the amount of glare for the user. With respect to the plan of treatment preparation, the user can adjust the fluence depending of the distance to the center of retina for taking into account that the size of the spot increases when you move towards the periphery and the system will automatically put the laser spot close or on the desired vessels.

In yet another related embodiment, with respect to eye safety, small eye movements are addressed by an eye tracking module which compensates for same through scanner displacement without blocking or impeding the treatment. In treating other eye pathologies using a camera that captures both camera images from each eye, due to the stereoscopic recombination algorithm taught herein, the user can obtain information on the depth (retinal detachment, indentation...). Multiple lens types could also be used such as a lens for periphery (superquad 160...) or a lens for central area, however the images obtained could have different definition depending of the lens magnification. In yet another embodiment, an electronic micromanipulator added to the system will add a joystick and control electronically the scanner zero in order to allow physician to add an offset on the scanner position (as opposed to making adjustments to the slit lamp position).

In this example embodiment, the physician has the option to update the initial picture taken of the patient's retina by integrating a picture or image taken after the last laser shot and/or the actual position of the laser shot, thereby providing a traceability feature (TRACEABILITY 2680) to system 2600 or 2000. Currently, the total number of times a laser is fired and the parameters used are generally not recorded, only the area being treated is recorded on paper. With systems 1000, 2000 and 2600 (and other embodiments described herein), all information regarding the laser parameters and position are recorded numerically and referenced (or associated) with the retina image.

In this example embodiment, the physician also has the option to use the previous laser shot performed to go farther (or extend more locally) in the periphery from the point or spot of the last laser shot (EXTENDED LOCALIZATION 2690 feature). The extended localization capability of the diagnostic/treatment systems described herein assists the physician to find his "bearings" within the periphery region that extends radially away from the macula or central portion of the retina since the periphery does not provide features or landmarks that a physician can recognize to more effectively navigate in this part of the eye. Images in this part of the eye may also be less clear hence the mosaic imaging function will not always work every time. As such, for example, the first part of the periphery (close to the central view) is first constructed through the MOSAIC function and then treated with the laser. The laser shots are visible on the image allowing updates of the retina image with these landmarks or previous markings present. The physician can now go farther in the periphery, equipped with part of the previously treated area in the current image available to him, thereby allowing the mosaic imaging function to identify the laser spot and integrate that new image (and area or part) of the far periphery into the global image. Without the presence of laser landmark (or part indicating the previously treated area), this part could not be integrated into the global image. In one example embodiment, the systems described herein are used to create mosaic images of the central region of the retina which aid in identifying which areas are to: 1) be treated; 2) be excluded; or 3) have the laser disarmed or temporarily halted if the current position is found on the mosaic image.

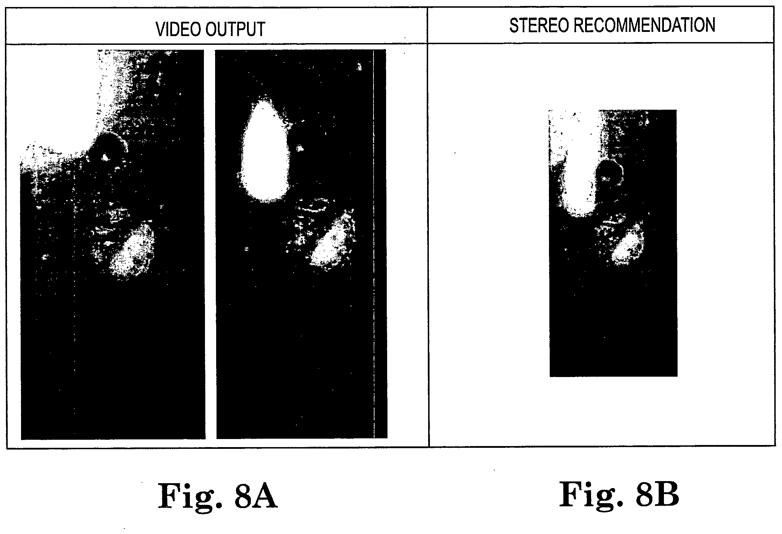

Referring again to FIGS. 5-7 and FIGS. 8A-8B, systems 1000, 2000 and 2600 provide a software function as part of the video imaging function of video module 2150 referred to as Video pretreatment and Stereo acquisition. In this example embodiment, two video cameras are located on each binocular optical viewing lens (right eye and left eyes of the physician) since the physician is rarely or ever focused on his two eyes at the same time. Each person generally has a preferred eye (left or right) for which the image is more focused, the second eye is not necessary for the image to be focused. Hence in this case, system 2000 (for example) will automatically recognize the side which is focused and will use it. If the second side is not focused, the system will not use the picture coming from that particular camera. The angle of view of each side of the binocular lens could be a little bit different which allows a viewing of a larger part of the retina. Some aspects or elements, such as the slit lamp reflection, are not in the same position on the two pictures. By recombining the two pictures of the video output via stereo recombination, the impact or effect of some elements can be reduced.

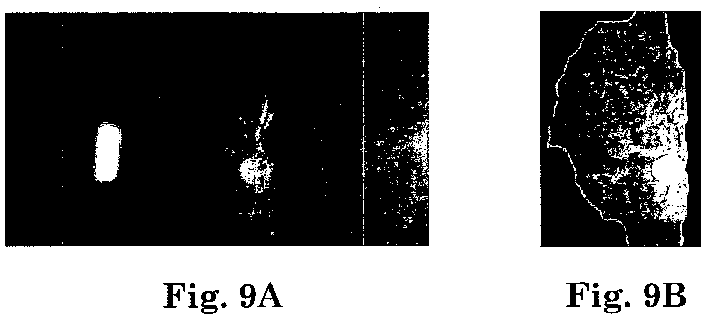

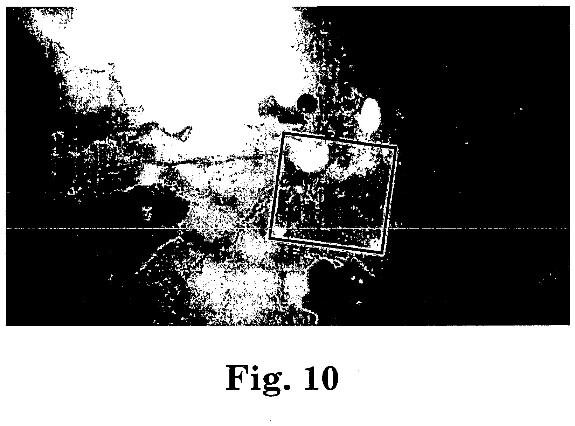

Another capability of video module 2150 is slit lamp extraction wherein a number of images filters will be implemented to extract only the useful area from the whole images taken of the retina: Histogram correction, deleting high intensity (saturated) area and low intensity area, recognition of red channel of the image are used to refine an original image to a final image (see FIGS. 9A-9B). Video module 2150 also provides the capability of image distortion characterization due to use of the aiming beam in the diagnostic or treatment system described herein. The portions of the image causing the image distortion can be modified using one or more of the following: different lenses, recognition that the retina of a patient could be more flat while another is more round, and during the step of acquiring the image using a pattern of aiming beam spots to characterize the deformation of the image in order to calibrate it or to correct it. In one example embodiment, 4 spots have been fired on the retinal tissue but the upper right spot is slightly to the left due to image distortion (see FIG. 10). In this example embodiment, the rectangle with the dotted line is the preset pattern without distortion while the solid line is the pattern with distortion.

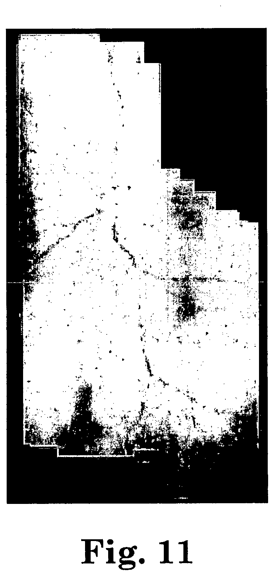

In building a mosaic image of the retina, even if only in part, it is important that the physician recognize that the imaging recording device will record what the physician sees when he moves about the patient's retina for the diagnosis and for mapping out the treatment. Systems 1000 or 2000, will first prompt the physician to perform a diagnostic of the retina. Simultaneously with the physician's diagnostic retinal review, the systems described herein will build in real time (via the system's computer) the mosaic image based on the pictures or images taken by the physician's video camera (see FIG. 11). In one example embodiment, the mosaic image could be partial or total as it we will not be necessary to create a mosaic image of the entire retina, only the area that is to be treated. The images added could vary as to form, size, distortion and zoom, so the software functions of the computer that will allow the reconstruction of the retina and will be sufficiently robust to authorize modification of the images in order to integrate them on the mosaic image. The mosaic image will be built in real time indicating to the user what area(s) of the eye that he has already passed or treated. The same part on the retina could be seen by a different position of the slit lamp (with the contact lens being more or less tilted), which may or may not lead to a distorted image.

In an example embodiment in which an aiming beam is part of the treatment system, the aiming beam is used to assist in administering the laser treatment as the size of the aiming beam spot(s) is an indication of the focus. In other example embodiments, the size of the spot pattern is an indication of the percentage of magnification zoom being used. In a related example embodiment in which an aiming beam is part of the diagnosis system, the aiming beam is used to calibrate the distortion for building the mosaic of the image. The distances between spots within the pattern is a calibration means of the amount of image distortion in order to correct the image.

Even though the main diagnostic tool for an ophthalmologist today still appears to be the slit lamp, one of its major weaknesses is the inability to record accurately a global image of the retina. An advantage of the diagnostic and treatment systems described herein, is the ability to build a retinal image or a mosaic image primarily using a slit lamp as well as the capability of having a retinal image that extends far into the periphery. Prior art diagnostic devices have been able to go to 45° and with the aid of recombination of multiples pictures only to 80° or 100°. Another prior art device appears to go farther into the periphery and provides an image at 200°, but it is not a live image (appears to use Scanner Lasing Ophthalmoscope (SLO) technique to acquire the image).

In another example embodiment, a GUI interface is provided in the diagnostic/treatment system described herein so that the physician has useful patient data on a screen that is close to the binocular vision or slit lamp viewer. The position of such a screen relative to the slit lamp is either fixed or movable from one side to the other of the slit lamp. For example, the current image of the slit lamp will be displayed on the left of the screen when the computer screen is on the right of the slit lamp, and displayed on the right when the screen is on the left of the slit lamp. Hence, the systems described herein are used at different times or intervals to record the evolution of a pathology or disease state. Some areas are identified while others ones are modified (negatively or positively) either due to the severity or complexity of the disease state or because the tissue is responding better than expected to the treatment. Due to the recorded superimposed images feature of the present invention, finding and characterizing these differences are much easier for the physician to detect. Further, during the diagnosis step, the physician can add annotation or comments to the images that can come up during the treatment phase.

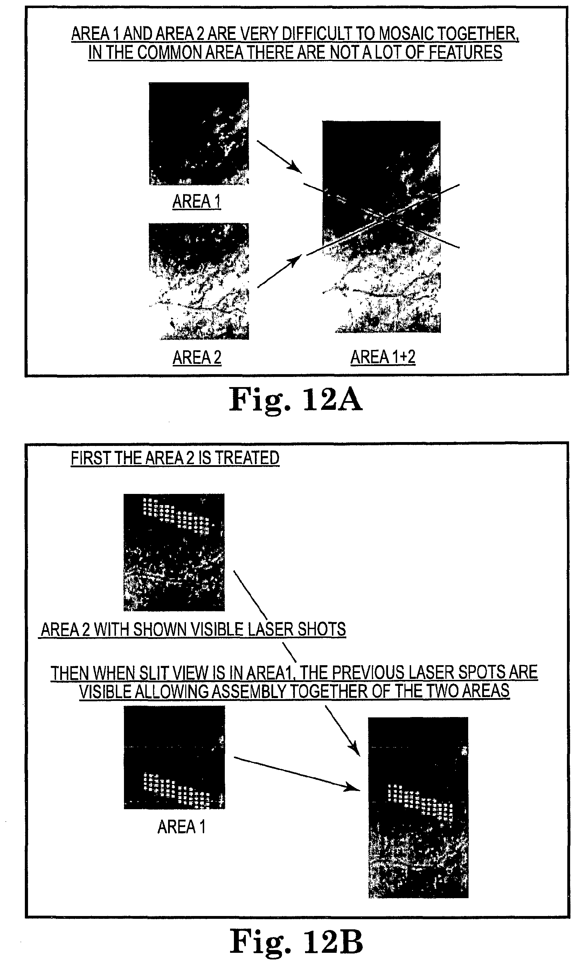

Referring now to FIGS. 12A-12B, these figures illustrate photographs of a retina periphery with visible laser shots (which are then joined together to form a mosaic image) for use in extended localization diagnosis or treatment according to the teachings of the invention. For the periphery it could be also possible to use the previous laser treatment (visible by a little white trace just after the laser shot) to localize the position of the slit lamp. In that case the reference image used to localize the current position is updated after each laser shot.

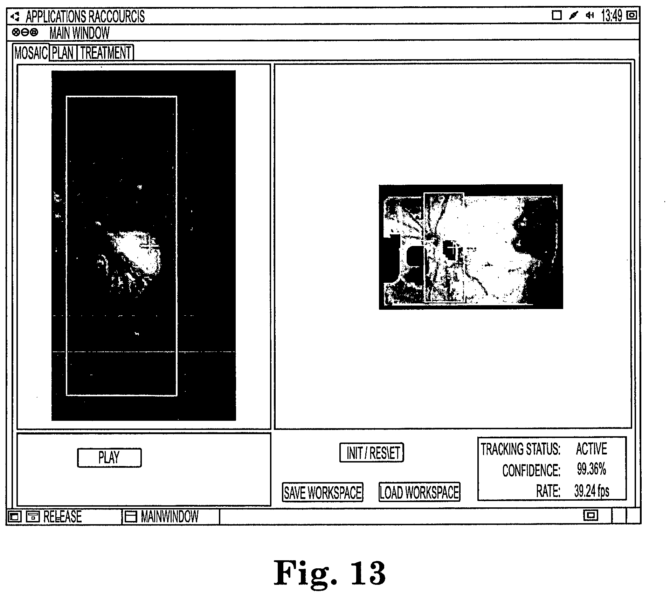

Referring now to FIGS. 13-17, there is illustrated a series of screenshots from the diagnostic and treatment system and software module, in particular they illustrate the various steps in the diagnostic and treatment methods that are taught herein. More particularly FIG. 13 illustrates an example of a screenshot from the software for creating a reference or mosaic image. On the left side is a view from a slit lamp of part of the patient's retina with a white upright rectangle serving as an image "cursor" or "paintbrush". The view of the right illustrates the reference or mosaic image being created right before the user's eyes as the white rectangle travels across the retina in all directions. The mosaic image on the right is being created in real time by the user.

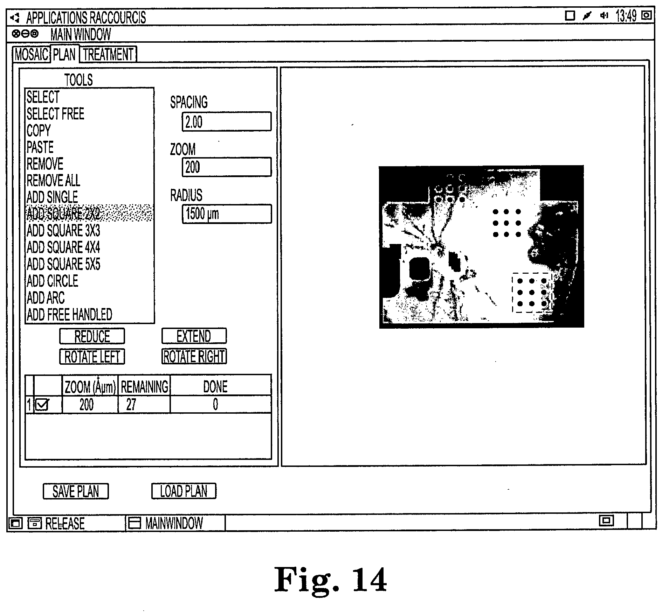

Referring now to FIG. 14, there is illustrated a screenshot of the method of preparing a treatment plan using a standard treatment pattern (such as a cube or rectangle with a plurality of spots). Once the mosaic image is formed in the above step, the physician or user moves the cursor about to select where on the retina image and what treatment pattern to use and, in this case, how many treatment patterns before actual treatment is performed.

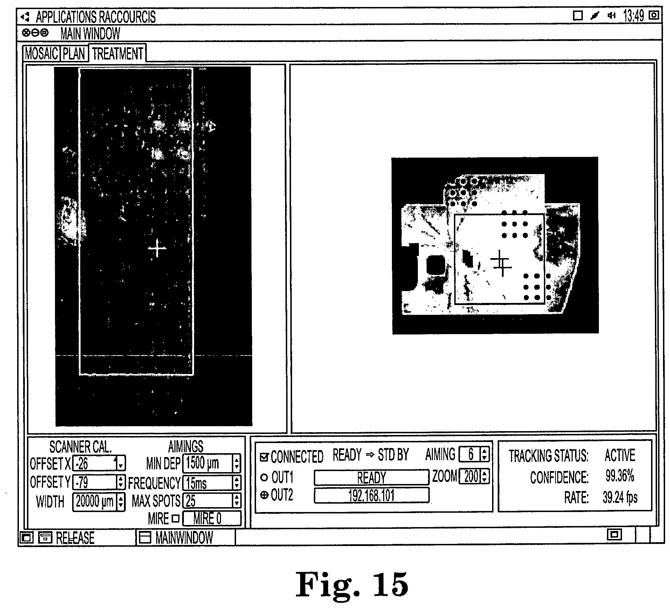

Referring now to FIG. 15, there is illustrated a screenshot of a laser treatment plan according to the teachings herein. In this step, the rectangle on the left first shows the areas to be treated as per the treatment plan by first showing the treatment patterns with the aiming beam. The view of the shows the aiming beam pattern with respect to the entire mosaic or reference image. Hence, all of the intended treatment steps are first shown with the aiming beam to assure the physician that this is the correct treatment plan before the treatment beam is fired. By way of example, the intended treatment area is first seen live on the screen in red with the aiming beam as the "proposed treatment" is fired the spots in the pattern turn green on the view on the right while the other patterns are still in red.

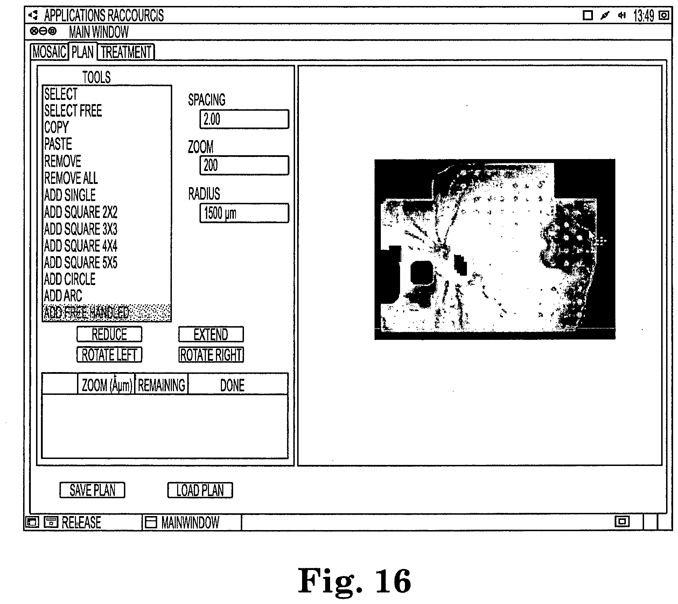

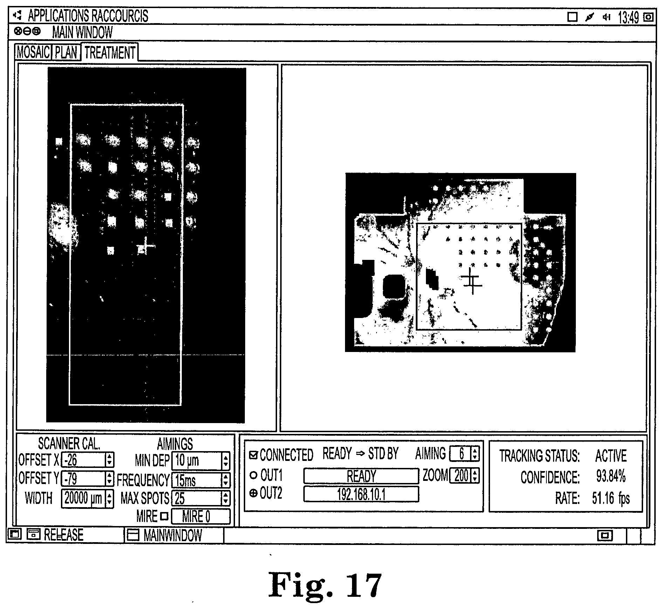

Referring now to FIG. 16, there is illustrated a screenshot of a laser treatment plan allowing a user to select a free area grid instead of a standard pattern. By way of example, a small 3 X 3 square of spots are used to fill in an area that is outlined or traced by the cursor. The view on the right shows the area being traced by the cursor and then filled in with the plurality of spots illustrating how the free area will be treated. Referring now to FIG. 17, there is illustrated a screenshot of a laser treatment on the free area or free formed grid of FIG. 16 according to a treatment plan. Laser treatment is then performed on that free-formed grid area. In this example, the differing colors in the spots of the pattern serve to indicate which spots have been treated and which are left to be treated.

One of the advantages of the aforementioned embodiment, is the capability of the diagnostic/treatment system is to project the aiming beam pattern of spots all at once instead of relying on the speed of the scanner to trace all of the spots of the pattern sufficiently fast so as to appear to the human eye as a single aiming beam pattern as in the prior art. With the teachings herein, the physician can overlay images of the proposed treatment pattern on live images of the retina and have a simulated run-through of the treatment without firing the laser. With this system we will automatically fill a large area selected by the physician with a grid or pattern of spots. Further, if the user prefers to continue with the pattern approach, the system can be configured to not energize the aiming beam of the whole pattern but only on the spot (or spots) localized or centered on the retina. This means that only a part of the pattern would be on and visible.

In various embodiments disclosed herein, the diagnostic/treatment system: creates an image of the retina, including the far periphery made only from the slit lamp; localizes the current slit lamp position in a global image of the retina; places a reference randomized laser spot in a treatment plan and not only a mono spot or predefined pattern; indicates with the aiming beam the position of the laser spot that will be delivered even if those spots are not in a predefined pattern; records the position of the delivered laser spot(s) even if it or they are not visible; calibrates the distortion of the retina image due to the aiming beam device; and uses the laser spot delivered to extend/expand and update the image of the retina.

The following patents and publications that relate to ophthalmology diagnostic and treatment systems are herein incorporated by reference in their entirety and constitute part of the disclosure herein: U.S. Patent and Publication Nos. 5,912,720; 6,454,410; 2001/0077625; 2012/016905; 2012/0165906; 2012/0239015; and 2012/0274901.

While the invention has been described above in terms of specific embodiments, it is to be understood that the invention is not limited to these disclosed embodiments. Upon reading the teachings of this disclosure many modifications and other embodiments of the invention will come to mind of those skilled in the art to which this invention pertains, and which are intended to be and are covered by both this disclosure and the appended claims. It is indeed intended that the scope of the invention should be determined by proper interpretation and construction of the appended claims and their legal equivalents, as understood by those of skill in the art relying upon the disclosure in this specification and the attached drawings. The foregoing specific embodiments of the invention as set forth in the specification herein are for illustrative purposes only. Various deviations and modifications may be made within the spirit and scope of the invention without departing from the main theme thereof.

## Claims

1. A system (1000, 2600) for generating ophthalmic images in real time for the treatment of eye diseases comprising:
- an eye imaging device (1100, 2100) which acquires a real-time image of a retina (10) of a patient (1500) for visualization by a user (1600);
- a video image module (1150, 2150) operatively coupled to said eye imaging device, said video image module including a video capturing device (2152) and a video processing unit (2154), the video capturing device adapted to capture real-time video images of the retina;
- an image processing module (2200) operatively coupled to said video image module (2150) and configured to generate a reference image of the retina from the real-time video images of the retina;
**characterized in that** the system comprises:
- an image recombination module (2300) operatively coupled to the image processing module (2200) and configured to overlay at least one secondary image received from at least one secondary ophthalmic diagnostic device(2350) over the reference image, thereby generating an augmented reality image;
- means for generating an ophthalmic treatment plan (2500) for the patient operatively coupled to said image recombination module (2300);
- a laser system (1300) and scanning device (1250) that are localized and centered in response to the treatment plan; and
- means for localizing and centering said eye imaging device in real time based on the reference image of the retina, said localizing means operatively coupled to said image processing module,
wherein the image processing module is configured to have a traceability function such that a position of a laser shot is updated on the treatment plan and the treatment plan is aligned with the reference image of the patient.

2. The system as in claim 1, wherein the video processing unit (2154) is configured to perform at least one of the operations from a group consisting of slit extraction, slit localization or centering, image reflection correction, image validation and image selection from at least one camera.

3. The system as in claim 1, wherein said reference image is selected from the group consisting of a mosaic image, an image from the secondary imaging device and a secondary ophthalmic diagnostic device.

4. The system as in claim 1, wherein at least one secondary image is selected from the group consisting previously stored photographic, retinographic, angiographic and OCT angiographic images.

5. The system as in claim 1, wherein said image recombination module (2300) is configured to overlay at least one secondary image from the secondary diagnostic device on a live slit lamp image of the patient thereby generating an augmented reality image.

6. The system as in claim 1, wherein the laser system and scanning device are adapted to be controlled in response to the augmented reality image and are localized as part of the treatment plan.

7. The system as in claim 1, further comprising an aiming beam adapted for calibrating the reference image that is being generated.

8. The system as in claim 1, wherein the image processing module (2200) is configured to have an extended localization function such that the reference image of the patient is updated with an image of the retina after a previous laser treatment to extend the reference image.

9. The system as in claim 1, wherein the system comprises:
a traceability module operatively coupled to the video module and adapted to update a retinal image of the patient, the retinal image update selected from the group consisting of an image of the retina after a previous laser firing, an updated position of the laser shot on the retinal image and an updated position of the laser.

10. The system of claim 9, further comprising localization means operatively coupled to said traceability module and adapted to extend the traceability function to a larger image, wherein the reference image is a mosaic image.

11. The system as in claim 1, further comprising an aiming beam module including an aiming beam operatively coupled to the image processing module and adapted to project at least one spot on the retina and to display to a user a pattern of spots on either a live retina image or the reference image.

12. The system of claim 11, wherein the aiming beam module is configured to fill a free formed area on the retinal image with one or more spots prior to a treatment.

13. The system of claim 11, wherein the aiming beam module is configured to not to energize the aiming beam of the entire pattern of spots but only on at least one spot localized or centered on the retina prior to a treatment.

14. The system of claim 11, wherein the aiming beam module is configured to provide at least one spot within the pattern of spots defined as an untreated spot, said untreated spot being defined to the user by a signal selected from the group consisting of a blinking spot, a spot of a different color, a spot of changing colors, and a spot of varying dimension or size or shape.

## Patentansprüche

1. System (1000, 2600) zum Erzeugen von Augenabbildungen in Echtzeit zur Behandlung von Augenerkrankungen, das Folgendes umfasst:
- eine Augenabbildungsvorrichtung (1100, 2100), die ein Echtzeitbild einer Retina (10) eines Patienten (1500) zur Visualisierung durch einen Benutzer (1600) erfasst;
- ein Videobildmodul (1150, 2150), das betrieblich mit der Augenabbildungsvorrichtung gekoppelt ist, wobei das Videobildmodul eine Videoaufnahmevorrichtung (2152) und eine Videoverarbeitungseinheit (2154) aufweist, wobei die Videoaufnahmevorrichtung angepasst ist, um Echtzeitvideobilder der Retina aufzunehmen;
- ein Bildverarbeitungsmodul (2200), das betrieblich mit dem Videobildmodul (2150) gekoppelt und konfiguriert ist, um ein Referenzbild der Retina aus den Echtzeitvideobildern der Retina zu erzeugen;
**dadurch gekennzeichnet, dass** das System Folgendes umfasst:
- ein Bildrekombinationsmodul (2300), das betrieblich mit dem Bildverarbeitungsmodul (2200) gekoppelt und konfiguriert ist, um mindestens ein Sekundärbild, das von mindestens einer sekundären ophthalmischen Diagnosevorrichtung (2350) empfangen wird, über das Referenzbild zu überlagern, wodurch ein Bild mit erweiterter Realität erzeugt wird;
- Mittel zum Erzeugen eines ophthalmischen Behandlungsplans (2500) für den Patienten, die betrieblich mit dem Bildrekombinationsmodul (2300) gekoppelt sind;
- ein Lasersystem (1300) und eine Scanvorrichtung (1250), die als Reaktion auf den Behandlungsplan lokalisiert und zentriert werden; und
- Mittel zum Lokalisieren und Zentrieren der Augenabbildungsvorrichtung in Echtzeit basierend auf dem Referenzbild der Retina, wobei die Lokalisierungsmittel betrieblich mit dem Bildverarbeitungsmodul gekoppelt sind,
wobei das Bildverarbeitungsmodul konfiguriert ist, um eine Rückverfolgbarkeitsfunktion derart aufzuweisen, dass eine Position eines Laserschusses auf dem Behandlungsplan aktualisiert wird und der Behandlungsplan mit dem Referenzbild des Patienten ausgerichtet wird.

2. System nach Anspruch 1, wobei die Videoverarbeitungseinheit (2154) konfiguriert ist, um mindestens einen der Vorgänge aus einer Gruppe auszuführen, die aus Spaltextraktion, Spaltlokalisierung oder -zentrierung, Bildreflexionskorrektur, Bildbestätigung und Bildauswahl aus der mindestens einen Kamera besteht.

3. System nach Anspruch 1, wobei das Referenzbild aus der Gruppe ausgewählt wird, die aus einem Mosaikbild, einem Bild aus der sekundären Abbildungsvorrichtung und einer zweiten ophthalmischen Diagnosevorrichtung besteht.

4. System nach Anspruch 1, wobei mindestens ein Sekundärbild aus der Gruppe ausgewählt wird, die aus vorab gespeicherten fotografischen, retinografischen, angiografischen und angiografischen OCT-Bildern besteht.

5. System nach Anspruch 1, wobei das Bildrekombinationsmodul (2300) konfiguriert ist, um mindestens ein Sekundärbild aus der sekundären Diagnosevorrichtung auf einem Live-Spaltlampenbild des Patienten zu überlagern, wodurch ein Bild mit erweiterter Realität erzeugt wird.

6. System nach Anspruch 1, wobei das Lasersystem und die Scanvorrichtung angepasst sind, um als Reaktion auf das Bild mit erweiterter Realität gesteuert zu werden und als Teil des Behandlungsplans lokalisiert sind.

7. System nach Anspruch 1, das ferner einen Zielstrahl umfasst, der angepasst ist, um das Referenzbild, das erzeugt wird, zu kalibrieren.

8. System nach Anspruch 1, wobei das Bildverarbeitungsmodul (2200) konfiguriert ist, um eine erweiterte Lokalisierungsfunktion derart aufzuweisen, dass das Referenzbild des Patienten mit einem Bild der Retina nach einer vorhergehenden Laserbehandlung aktualisiert wird, um das Referenzbild zu erweitern.

9. System nach Anspruch 1, wobei das System Folgendes umfasst:
ein Rückverfolgbarkeitsmodul, das mit dem Videomodul betrieblich gekoppelt und angepasst ist, um ein Retinabild des Patienten zu aktualisieren, wobei die Retinabildaktualisierung aus der Gruppe ausgewählt wird, die aus einem Bild der Retina nach einem vorhergehenden Laserschuss, einer aktualisierten Position des Laserschusses auf dem Retinabild und einer aktualisierten Position des Lasers ausgewählt wird.

10. System nach Anspruch 9, das ferner Lokalisierungsmittel umfasst, die betrieblich mit dem Rückverfolgungsmodul gekoppelt und angepasst sind, um die Rückverfolgungsfunktion auf ein größeres Bild zu erweitern, wobei das Referenzbild ein Mosaikbild ist.

11. System nach Anspruch 1, das ferner ein Zielstrahlmodul umfasst, das einen Zielstrahl aufweist, der betrieblich mit dem Bildverarbeitungsmodul gekoppelt und angepasst ist, um mindestens einen Fleck auf die Retina zu projizieren und einem Benutzer ein Muster aus Flecken auf entweder einem Live-Retinabild oder dem Referenzbild anzuzeigen.

12. System nach Anspruch 11, wobei das Zielstrahlmodul konfiguriert ist, um eine frei gebildete Fläche auf dem Retinabild mit einem oder mehreren Flecken vor einer Behandlung zu füllen.

13. System nach Anspruch 11, wobei das Zielstrahlmodul konfiguriert ist, um den Zielstrahl des gesamten Musters aus Flecken nicht zu erregen, sondern nur mindestens einen Fleck, der vor einer Behandlung auf der Retina lokalisiert oder zentriert wird.

14. System nach Anspruch 11, wobei das Zielstrahlmodul konfiguriert ist, um mindestens einen Fleck innerhalb des Musters von Flecken bereitzustellen, der als ein unbehandelter Fleck definiert ist, wobei der unbehandelte Fleck zu dem Benutzer durch ein Signal definiert wird, das aus der Gruppe ausgewählt wird, die aus einem blinkenden Fleck, einem Fleck mit unterschiedlicher Farbe, einem Fleck mit wechselnden Farben und einem Fleck mit variierenden Maß oder variierender Größe oder Form besteht.

## Revendications

1. Système (1000, 2600) pour générer des images ophtalmiques en temps réel pour le traitement de maladies oculaires comprenant :
- un dispositif d'imagerie de l'oeil (1100, 2100) qui acquiert une image en temps réel d'une rétine (10) d'un patient (1500) pour visualisation par un utilisateur (1600) ;
- un module d'image vidéo (1150, 2150) couplé fonctionnellement audit dispositif d'imagerie de l'oeil, ledit module d'image vidéo incluant un dispositif de capture vidéo (2152) et une unité de traitement vidéo (2154), le dispositif de capture vidéo étant adapté pour capturer des images vidéo en temps réel de la rétine ;
- un module de traitement d'image (2200) couplé fonctionnellement audit module d'image vidéo (2150) et configuré pour générer une image de référence de la rétine à partir des images vidéo en temps réel de la rétine ;
**caractérisé en ce que** le système comprend :
- un module de recombinaison d'image (2300) couplé fonctionnellement au module de traitement d'image (2200) et configuré pour recouvrir au moins une image secondaire reçue à partir d'au moins un dispositif de diagnostic ophtalmique secondaire (2350) sur l'image de référence, générant ce faisant une image à réalité augmentée ;
- des moyens de génération d'un plan de traitement ophtalmique (2500) pour le patient couplés fonctionnellement audit module de recombinaison d'image (2300) ;
- un système laser (1300) et un dispositif de balayage (1250) qui sont localisés et centrés en réponse au plan de traitement ; et
- des moyens de localisation et de centrage dudit dispositif d'imagerie de l'oeil en temps réel sur la base de l'image de référence de la rétine, lesdits moyens de localisation étant couplés fonctionnellement audit module de traitement d'image,
dans lequel le module de traitement d'image est configuré pour avoir une fonction de traçabilité de sorte qu'une position d'un tir laser est mise à jour sur le plan de traitement et le plan de traitement est aligné avec l'image de référence du patient.

2. Système selon la revendication 1, dans lequel l'unité de traitement vidéo (2154) est configurée pour réaliser au moins une des opérations d'un groupe consistant en l'extraction par fente, la localisation ou le centrage de fente, la correction de la réflexion d'image, la validation d'image et la sélection d'image à partir d'au moins une caméra.

3. Système selon la revendication 1, dans lequel ladite image de référence est sélectionnée dans le groupe consistant en une image en mosaïque, une image provenant du dispositif d'imagerie secondaire et d'un dispositif de diagnostic ophtalmique secondaire.

4. Système selon la revendication 1, dans lequel au moins une image secondaire est sélectionnée dans le groupe consistant en des images photographiques, rétinographiques, angiographiques et angiographiques OCT stockées précédemment.

5. Système selon la revendication 1, dans lequel ledit module de recombinaison d'image (2300) est configuré pour recouvrir au moins une image secondaire du dispositif de diagnostic secondaire sur une image de lampe à fente en direct du patient générant ce faisant une image à réalité augmentée.

6. Système selon la revendication 1, dans lequel le système laser et le dispositif de balayage sont adaptés pour être commandés en réponse à l'image à réalité augmentée et sont localisés en tant que partie du plan de traitement.

7. Système selon la revendication 1, comprenant en outre un faisceau de visée adapté pour le calibrage de l'image de référence qui est générée.

8. Système selon la revendication 1, dans lequel le module de traitement d'image (2200) est configuré pour avoir une fonction de localisation étendue de sorte que l'image de référence du patient est mise à jour avec une image de la rétine après un traitement laser précédent pour étendre l'image de référence.

9. Système selon la revendication 1, dans lequel le système comprend :
un module de traçabilité couplé fonctionnellement au module vidéo et adapté pour mettre à jour une image rétinienne du patient, la mise à jour d'image rétinienne étant sélectionnée dans le groupe consistant en une image de la rétine après un déclenchement laser précédent, une position mise à jour du tir laser sur l'image rétinienne et une position mise à jour du laser.

10. Système selon la revendication 9, comprenant en outre des moyens de localisation couplés fonctionnellement audit module de traçabilité et adaptés pour étendre la fonction de traçabilité pour une image plus grande, dans laquelle l'image de référence est une image en mosaïque.

11. Système selon la revendication 1, comprenant un module de faisceau de visée incluant un faisceau de visée couplé fonctionnellement au module de traitement d'image et adapté pour projeter au moins un point sur la rétine et pour afficher à un utilisateur un motif de points sur soit une image de rétine en direct soit l'image de référence.

12. Système selon la revendication 11, dans lequel le module de faisceau de visée est configuré pour remplir une zone de forme libre sur l'image rétinienne avec un ou plusieurs points avant un traitement.

13. Système selon la revendication 11, dans lequel le module de faisceau de visée est configuré pour ne pas exciter le faisceau de visée de l'ensemble du motif de points mais seulement sur un au moins un point localisé ou centré sur la rétine avant un traitement.

14. Système selon la revendication 11, dans lequel le module de faisceau de visée est configuré pour fournir au moins un point à l'intérieur du motif de points défini en tant que point non traité, ledit point non traité étant défini pour l'utilisateur par un signal sélectionné dans le groupe consistant en un point clignotant, un point d'une couleur différence, un point de couleurs changeantes, et un point de dimension ou de taille ou de forme variable.
